# EUROPEAN PATENT SPECIFICATION

(11) **EP 1 169 456 B1**
(45) Date of publication and mention of the grant of the patent: **11.07.2012**
(21) Application number: 00931926.0
(22) Date of filing: 10.04.2000
(51) Int. Cl.: C07K 14/33, C12N 15/73, C12P 21/02, A61K 39/08

(54) **Recombinant production of Clostridium difficile Toxin A or Toxin B**
Rekombinante Herstellung vom Toxin A oder Toxin B der Clostridium difficile
Production recombinante de Toxine A ou Toxine B de Clostridium difficile

(30) Priority: 09.04.1999 US 128686 P; 01.03.2000 US 186201 P; 20.03.2000 US 190111 P
(43) Date of publication of application: 09.01.2002
(60) Divisional application: 12155470.3
(73) Proprietor: Intercell USA, Inc., Gaithersburg, MD 20878 (US)
(72) Inventor: WILKINS, Tracy, D., Riner, VA 24149 (US); LYLERLY, David, M., Radford, VA 24141 (US); MONCRIEF, J., Scott, Christiansburg, VA 24073 (US); ZHENG, Limin, Techlabs, Inc., Blacksburg, VA 24060-6364 (US); PHELPS, Carol, Floyd, VA24091 (US)
(74) Representative: Vossius & Partner
(86) International application number: PCT/US2000/009525
(87) International publication number: WO 2000/061762

(56) References cited:
- WO-A-96/12802
- WO-A-97/02836
- LYERLY ET AL: "vaccination against lethal Clostridium difficile Enterocolitis with a nontoxic recombinant peptide of toxin A", CURRENT MICROBIOLOGY, SPRINGER, NEW YORK, NY, US, vol. 21, 1 July 1990 (1990-07-01), pages 29-32, XP002083271, ISSN: 0343-8651, DOI: 10.1007/BF02090096

## Description

### TECHNICAL FIELD OF INVENTION

The present invention relates to the field of medical immunology and further to pharmaceutical compositions, methods of making and methods of use of vaccines. More specifically this invention relates to a method of producing recombinant proteins derived from the genes encoding *Clostridium difficile* toxin A and toxin B.

### BACKGROUND OF THE INVENTION

*Clostridium difficile,* a Gram positive anaerobic spore-forming bacillus is an etiologic agent of antibiotic associated diarrhea (AAD) and colitis (AAC). The symptoms of the disease range from mild diarrhea to fulminant and life-threatening pseudomembranous colitis (PMC). Antibiotic therapy can disrupt the normal intestinal microflora. Destruction of the normal flora results in a condition in which spores of *C. difficile* can germinate and the organism can grow and produce disease causing toxins. *C. difficile* causes about 25% of antibiotic-associated diarrheas, however, it is almost always the causative agent of PMC (Lyerly, D.M. and T.D. Wilkins, in Infections of the Gastrointestinal Tract, Chapter 58, pages 867-891, (Raven Press, Ltd, New York 1995)). Additionally, *C*. *difficile* is frequently identified as a causative agent of nosocomial infectious diarrheas, particularly in older or immuno-compromised patients (U.S. Pat. No. 4,863.852 (Wilkins et al.) (1989)).

Disease caused by *C*. *difficile* is due to two enteric toxins A and B produced by toxigenic strains (U.S. Pat. No. 5,098,826 (Wilkins et al.) (1992)). Toxin A is an enterotoxin with minimal cytotoxic activity, whereas toxin B is a potent cytotoxin but has limited enterotoxic activity. The extensive damage to the intestinal mucosa is attributable to the action of toxin A, however, toxins A and B act synergistically in the intestine.

The genetic sequences encoding both toxigenic proteins A and B, the largest known bacterial toxins, with molecular weights of 308,000 and 269.000. respectively, have been elucidated (Moncrief et al., Infect. Immun. 65:1105-1108 (1997); Barroso et al., Nucl. Acids Res. 18:4004 (1990); Dove et al. Infect. Immun. 58:480-488 (1990)). Because of the degree of similarity when conserved substitutions are considered, these toxins are thought to have arisen from gene duplication. The proteins share a number of similar structural features with one another. For example, both proteins possess a putative nucleotide binding site, a central hydrophobic region, four conserved cysteines and a long series of repeating units at their carboxyl ends. The repeating units of toxin A, particularly, are immunodominant and are responsible for binding to type 2 core carbohydrate antigens on the surface of the intestinal epithelium (Krivan et al.. Infect. Immun. 53:573-581 (1986); Tucker. K. and T.D. Wilkins, Infect. Immun. 59:73-78 (1991)).

The toxins share a similar molecular mechanism of action involving the covalent modification of Rho proteins. Rho proteins are small molecular weight effector proteins that have a number of cellular functions including maintaining the organization of the cytoskeleton. The covalent modification of Rho proteins is due to glucosyltransferase activity of the toxins. A glucose moiety is added to Rho using UDP-glucose as a co-substrate (Just et al. Nature 375:500-503 (1995), Just et al. J. Biol. Chem 270:13932-13939 (1995)). The glucosyltransferase activity has been localized to approximately the initial 25% of the amino acid sequence of each of these toxins (Hofmann et al. J. Biol. Chem. 272:11074-11078 (1997), Faust and Song, Biochem. Biophys. Res. Commun. 251:100-105 (1998)) leaving a large portion of the toxins, including the repeating units, that do not participate in the enzymatic activity responsible for cytotoxicity.

The toxin A protein comprises 31 contiguous repeating units ( *r*ARU ) and may contain multiple T cell epitopes (Dove et al. Infect. Immun. 58:480-488 (1990). The repeating units are defined as class I repeats and class II. *r*ARU may be uniquely suited for use in inducing T cell-dependent response to an antigen. The sequence of each unit is similar but not identical. These features along with its usefulness in eliciting toxin A neutralizing antibodies make *r*ARU a novel candidate as a carrier protein.

The toxin B repeating units have similar features to those of *r*ARU. Like rARU. the recombinant toxin B repeating units (rBRU) are relatively large (~70 kDa) and are composed of contiguous repeats of similar amino acid sequences (Barroso et al. Nucleic Acids Res. 18:4004 (1990); Eichel-Streiber et al. Gene 96:107-113 (1992)). Less is known about this portion of toxin B than the binding domain of toxin A.

Thomas et al (U.S. Pat. No. 5.919,463 (1999)) disclose C. *difficile* toxin A or toxin B or certain fragments thereof as mucosal adjuvants intranasally administered to stimulate an immune response to an antigen (e.g., Helicobacter pylori urease, ovalbumin (OVA), or keyhole limpet hemocyanin (KLH)). However, Thomas does not teach the use of such adjuvant for protection against strains of *C*. *difficile.* Lyerly et al. Current Microbiology 21:29-32 (1990) considered at a smaller recombinant fragment from the toxin A repeats in hamster protection assays. However, these data suggest at best only a very weak or partial protection from strains of *C*. *difficile*, whereas the present disclosure demonstrates the use of *C. difficile* toxin repeating units that provide a clear immunogenic response and at higher levels, which afford protection against *C*. *difficile.*

Even were one to consider *r*ARU and *r*BRU as candidate proteins for conjugate vaccines, the production of such proteins presents certain challenges. There are methods for the production of toxin A and antibodies elicited thereto (U.S. Pat. No. 4.530.833 (Wilkins et al.)(1985*);* U.S. Pat. No. 4,533.630 (Wilkins et al.)(1985); and U.S. Pat. No. 4,879,218 (Wilkins et al.)(1989); WO 9612802). There are significant difficulties in producing sufficient quantities of the *C*. *difficile* toxin A and toxin B proteins. These methods are generally cumbersome and expensive. However, the present invention provides for the recombinant expression of nontoxic truncated portions or fragments of *C*. *difficile* toxin A and toxin B in strains of *E*. *coli.* Such methods are more effective and commercially feasible for the production of sufficient quantities of a protein molecule for raising humoral immunogenicity to antigens.

Part of the difficulty that the present invention overcomes concerns the fact that large proteins are difficult to express at high levels in *E. coli.* Further, an unusually high content of AT in these clostridial gene sequences (i.e., AT-rich) makes them particularly difficult to express at high levels (Makoff et al. Bio/Technology 7:1043-1046 (1989)). It has been reported that expression difficulties are often encountered when large (i.e., greater than 100 kd) fragments are expressed in E. coli. A number of expression constructs containing smaller fragments of the toxin A gene have been constructed, to determine if small regions of the gene can be expressed to high levels without extensive protein degradation. In all cases, it was reported that higher levels of intact, full length fusion proteins were observed rather than the larger recombinant fragments (Kink et al., U.S. Pat. No. 5.736.139; see: Example 11(c)). It has been further reported that AT-rich genes contain rare codons that are thought to interfere with their high-level expression in *E. coli* (Makoff et al. Nucleic Acids Research 17:10191-10202). The present invention provides for methods to express genes that are both large and AT-rich and that encode rARU or rBRU or a fusion protein thereof. For example, the toxin A repeating units are approximately 98 kDa and the gene sequence has an AT content of approximately 70% that is far above the approximately 50% AT content of the *E. coli* genome. The present invention provides for methods of expressing AT-rich rARU or rBRu genes (including very large ones) at high levels in *E*. *coli* without changing the rare codons or supplying rare tRNA.

### SUMMARY OF THE INVENTION

The present invention is as characterized in the claims. The present disclosure describes an immunogenic composition that includes recombinant proteins. The genes encoding the proteins are isolated from a strain of *C*. *difficile.* The toxin is *C*. *difficile* toxin A or toxin B. The recombinant protein components are preferably nontoxic and comprise a portion of both toxins including all of the amino acid sequence of the *C*. *difficile* toxin A or toxin B repeating units (*r*ARU or *r*BRU) or fragment thereof. The described immunogenic composition may further include a carbohydrate moiety as well as a pharmaceutically acceptable carrier or other compositions in a formulation suitable for injection in a mammal.

It is also conceirable that the rARU and *r*BRU components are combined, preferably in a manner that results in high levels of neutralizing antibodies to toxins A and B when the immunogenic composition is used in vaccine. The components may be admixed at different ratios. Further, the rARU and rBRU components may be chemically or physically linked to form a complex. The *r*ARU and *r*BRU sequences, or fragments thereof, may be genetically fused in a manner that results in the production of a hybrid molecule. The immunogenic composition elicits antibodies that precipitate the native *C*. *difficile* toxins and neutralize their cytotoxic activity thus providing protection against *C*. *difficile* associated disease.

### BRIEF DESCRIPTION OF THE DRAWINGS

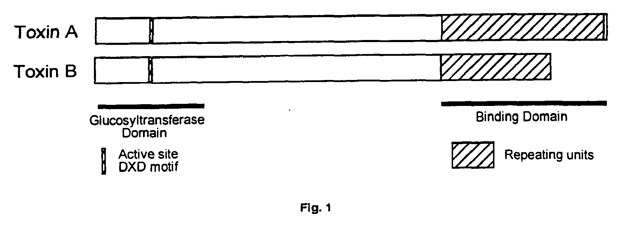

Fig. 1. Schematic of toxins A and B. The repeating units of the toxins function in binding to the cell surface. Antibodies to the repeating units of the toxins neutralize cytotoxic activity by blocking the binding of the toxins to the cell surface.

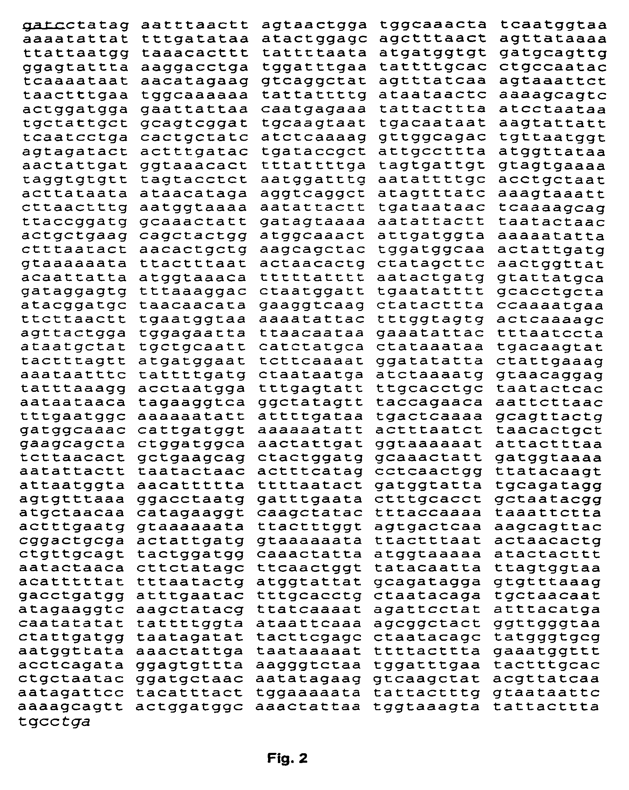

Fig. 2 shows the nucleotide sequence (numbers 5690-8293. GenBank accession number M30307. Dove *et al*. 1993) of the toxin A gene region that encodes rARU and the toxin A stop codon. The sequence encodes for the entire repeating units of toxin A from *C*. *difficile* strain VPI 10463 as defined by Dove *et al.* (Dove *et al*., *Infect Immun*. 58:480-488 8 (1990)). In addition it encodes for 4 amino acids upstream of the beginning of the repeating units and a small stretch of hydrophobic amino acids at the end of toxin A. The Sau3A site (underlined) at the beginning of the sequence was used to subclone the gene fragment to an expression vector. The stop codon at the end of the sequence is italicized.

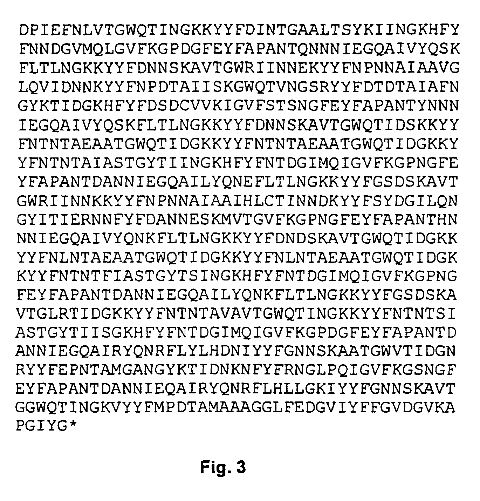

Fig. 3 shows the amino acid sequence (GenBank accession number M303307) of rARU. The disclosure contemplates the use of any recombinant protein containing this amino acid sequence, any fragment therein, any fusion protein containing *r*ARU or a fragment therein, and any larger fragment from toxin A carrying all or part of *r*ARU, as a carrier for conjugate vaccine compositions.

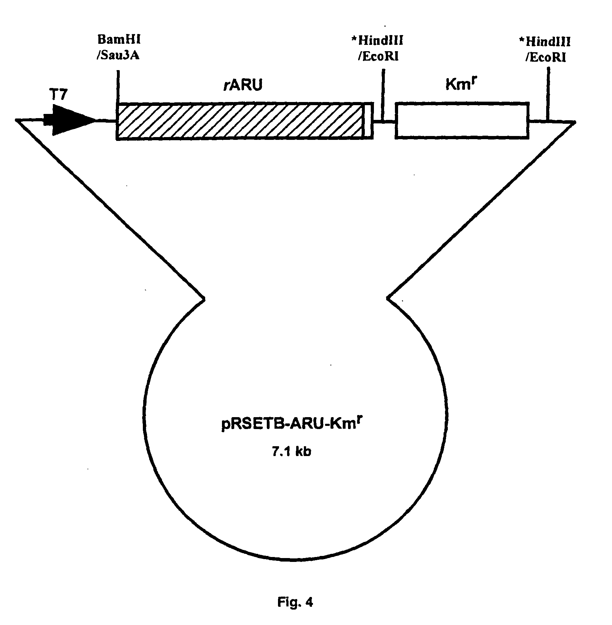

Fig. 4 shows the expression vector pRSETB-ARU-Km^{r} used for expression of *r*ARU. A Sau3A/HindIII gene fragment of approximately 2.7 kb containing the entire nucleotide sequence encoding *r*ARU. stop codon, and a small region downstream of the toxin A stop codon, was subcloned to the vector pRSETB digested with BamHI and HindIII. In a subsequent step the kanamycin resistance gene was subcloned at the HindIII site located downstream of the *r*ARU gene fragment. The 1.2 kb fragment encoding the Km^{r} gene was derived from pUC4K (GenBank accession number X06404) by digestion with EcoRI and subcloned at the HindIII site after blunt ending of the vector and Km^{r} cassette with Klenow fragment. Expression vector pRSETB-ARU-Km^{r} was transformed into BL21(DE3) for expression of *r*ARU under control of the T7 promoter.
   * HindIII/EcoRI sites were eliminated by blunt ending.

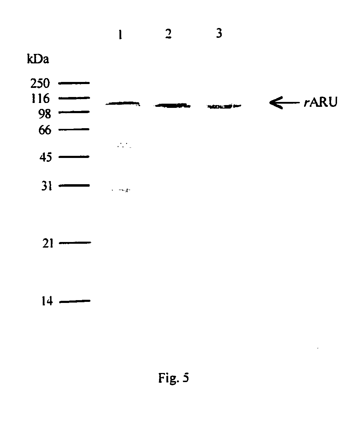

Fig. 5 shows an SDS-PAGE gel (15% acrylamide) ofrARU expression and purification steps. Lanes: 1) 4 µl of 10X BL21(DE3) *E*. *coli*/pRSETB-ARU-Km^{r} lysate 2) 4 µl of dialyzed 40% ammonium sulfate fraction at 10X relative to the original culture volume 3) 5 µl *r*ARU (0.88 mg/ml) purified by CL-6B Sepharose anion exchange chromatography.

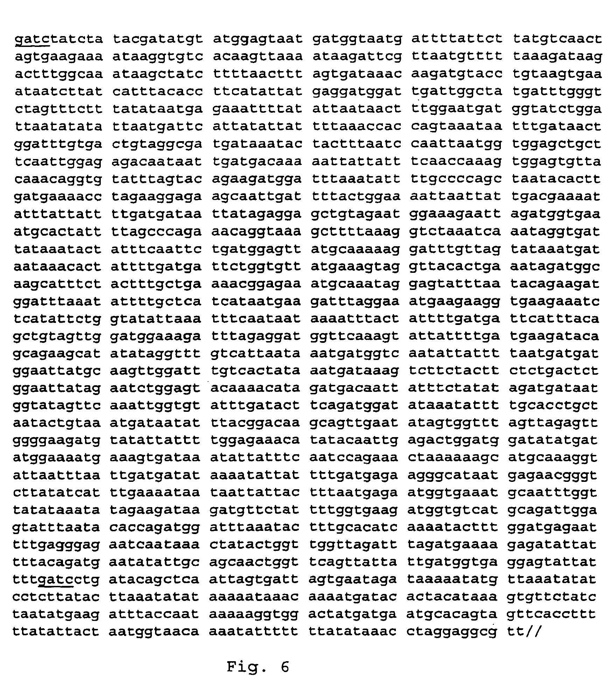

Fig. 6 shows the nucleotide sequence (GenBank accession number X531138, Wilkins *et al*. 1990) of the toxin B gene region that encodes rBRU and a small portion upstream. The Sau3a restriction sites used for subcloning are underlined. The sequence of the repeating units of toxin B from *C*. *difficile* strain VPI was defined previously (Eichel-Streiber et al. Mol. Gen. Gen. 233:260-268).

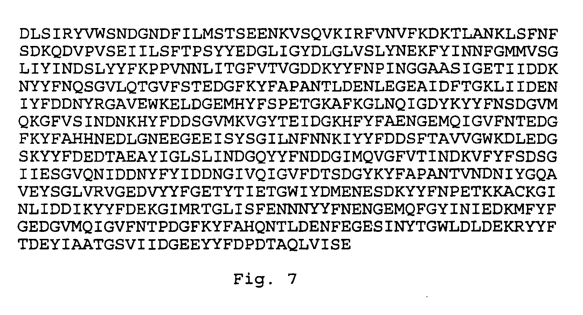

Fig. 7 shows the amino acid sequence (GenBank accession number X53138) of *r*BRU and a small upstream region. The disclosure contemplates the use of any recombinant protein containing this amino acid sequence, any fragment therein, any fusion protein containing *r*BRU or a fragment therein, and any larger fragment from toxin B carrying all or part of *r*BRU, as a component in a vaccine against *C*. *diflicile.*

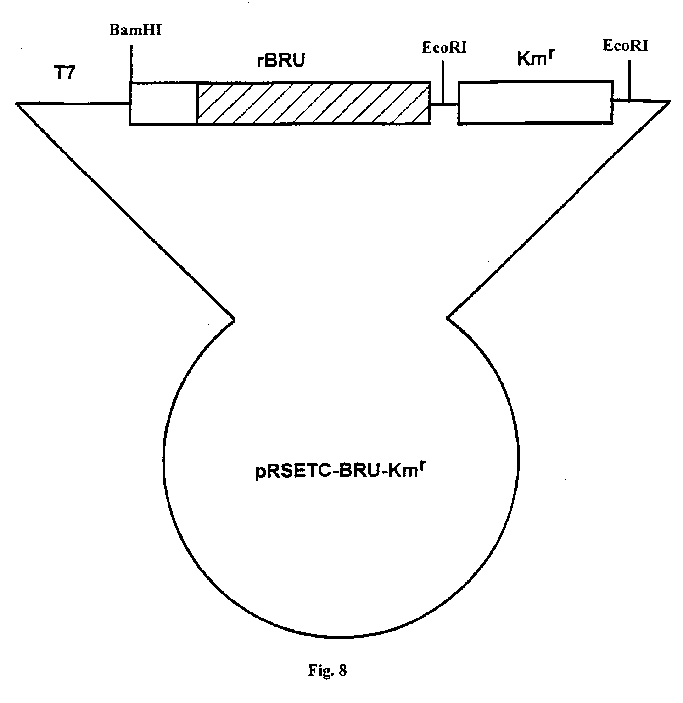

Fig. 8 shows the expression vector pRSETC-BRU-Km^{r} used for expression of *r*BRU. A gene fragment of approximately 1.8 kb containing nearly the entire nucleotide sequence encoding *r*BRU (final 10 amino acids of toxin B are eliminated) was subcloned from the toxin B gene (Phelps et al. Infect. Immun. 59:150-153 (1991)) to pGEX-3X. A BamHI/EcoRI from pGEX-3X-BRU was subcloned to pRSETC. In a subsequent step the kanamycin resistance gene was subcloned at the EcoRI site located downstream of the *r*BRU gene fragment. The 1.2 kb fragment encoding the Km^{r} gene was derived from pUC4K (GenBank accession number X06404) by digestion with EcoRI. Expression vector pRSETC-BRU-Km^{r} was transformed into BL21(DE3) for expression of *r*BRU under control of the T7 promoter.

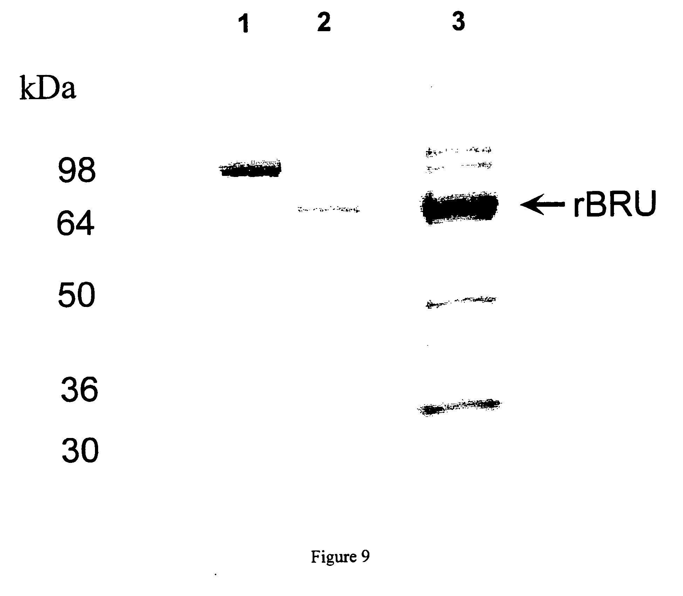

Fig. 9. SDS-PAGE of purified *r*ARU and partially purified rBRU. Lanes: 1) rARU purified by sequential ammonium sulfate precipitation and Sepharose CL-6B anion exchange chromatography, 2) rBRU partially purified by ammonium sulfate precipitation and hydrophobic interaction chromatography on phenyl Sepharose, 3) lysate (10X concentration) of *Escherichia coli* BL21 (DE3)/pRSETC-BRU-Km^{r}.

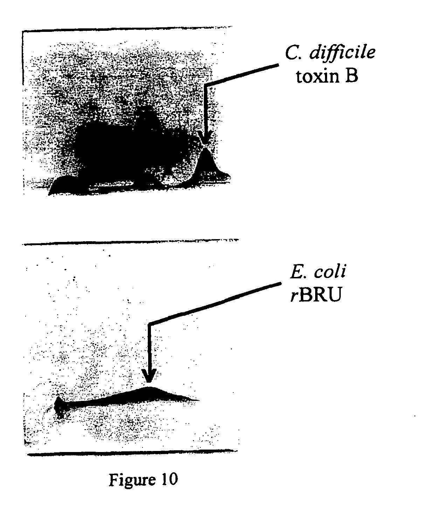

Fig. 10. Crossed-immunoelectrophoresis of (A) *C*. *difficile* culture filtrate and (B) partially purified *r*BRU. *C*. *difficile* goat antisera was used as the precipitating antibody.

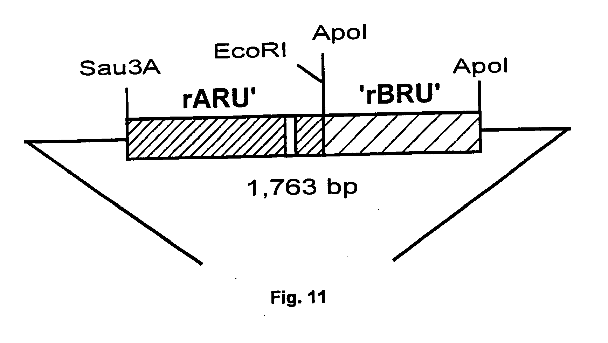

Fig. 11. shows an example of a genetic fusion of *r*ARU and *r*BRU. A Sau3A/EcoRI toxin A gene fragment (nucleotides 5530 through 6577) may be fused to an ApoI toxin B gene fragment (nucleotides 5464 through 6180) to create an in-frame 1,763 bp gene fusion expressing a 588 amino acid rARU'/'rBRU' fusion protein of approximately 68 kDa containing a significant portion of the repeating units from both toxin genes. The rARU' fragment encodes an epitope for PCG-4 represented by the open bar in the rARU' portion of the gene fusion. of the gene

### DETAILED DESCRIPTION OF THE INVENTION

The present invention is drawn to the methods to produce the repeating unit portion of clostridium difficile toxin A (rARU) or toxin B (rBRU) as defined in the claims. The disclosure further describes an immunogenic composition that includes at least one recombinant protein component, wherein the gene encoding the protein component is isolated from a strain of *Clostridium difficile* and wherein the toxin is toxin A, optionally a portion of the toxin containing all of the amino acid sequence of the toxin A repeating units (*r*ARU) or fragment thereof, or wherein the toxin is toxin B, optionally a portion of the toxin containing all of the amino acid sequence of the repeating units (*r*BRU) or a fragment thereof. The immunogenic composition may further include a pharmaceutically acceptable carrier or other compositions in a formulation suitable for injection in a mammal.

These described immunogenic compositions elicit an immune response in a mammalian host, including humans and other animals. The immune response may be either a cellular dependent response or an antibody dependent response or both and further the response may provide immunological memory or a booster effect or both in the mammalian host. The described immunogenic compositions are useful as vaccines and may provide a protective response by the mammalian subject or host to infection by strains of *Clostridium difficile.*

The present disclosure also describes methods for producing an immunogenic composition by: constructing a genetic sequence encoding a recombinant protein component, where the gene encoding the protein component is isolated from a strain of *Clostridium difficile*, expressing the recombinant protein component in a microbial host; recovering the recombinant protein from a culture of the host; conjugating the protein to a second protein component, and recovering the conjugated protein and polysaccharide component. The protein component may also consist of a fusion protein, whereby a portion of said recombinant protein is genetically fused to a second protein component. The expression of the genetic sequence in a method according to the invention is regulated by an inducible promoter that is operatively positioned upstream of the sequence and is functional in the host. Even further, said genetic sequence is maintained throughout the growth of the host by constant and stable selective pressure. Maintenance of the expression vector may be conferred by incorporation in the expression vector of a genetic sequence that encodes a selective genotype, the expression of which in the microbial host cell results in a selective phenotype. Such selective genotypes, include a gene encoding resistance to antibiotics, such as kanamycin. The expression of this selective genotypic sequence on the expression vector in the presence of a selective agent or condition, such as the presence of kanamycin, results in stable maintenance of the vector throughout growth of the host. A selective genotype sequence could also include a gene complementing a conditional lethal mutation.

Other genetic sequences may be incorporated in the expression vector, such as other drug resistance genes or genes that complement lethal mutations. The microbial host used in the method of this invention is *E*. *coli*.

The methods of the present invention also provide for a level of expression of the recombinant protein in the host at a level greater than about 50 mg/liter and even more preferably at 100 mg/liter or greater than about 100 mg/liter. The molecular weight of the protein is greater than about 30 kDa, preferably greater than about 50 kDa and even more preferably greater than about 90 kDa. This invention also provides that the protein may be recovered by any number of methods known to those in the art for the isolation and recovery of proteins, but preferably the recovery is by ammonium sulfate precipitation followed by ion exchange chromatography.

The present disclosure further describes methods for preparing the immunogenic composition that provides that the protein component is conjugated to a second protein component by one of a number of means known to those in the art, particularly an amidization reaction.

Also, high yields of recombinant protein may be dependent on the growth conditions, the rate of expression, and the length of time used to express AT-rich gene sequences. In general, AT-rich genes appear to be expressed at a higher level in E. *coli* during a post-exponential or slowed phase of growth. High-level production of the encoded protein requires moderate levels of expression over an extended period (e.g. 20-24 h) of post-exponential growth rather than the typical approach of high-level expression during exponential growth for shorter periods (e.g. 4-6 h). In this regard, it is more efficient to maintain plasmids carrying the gene of interest by maintaining constant selective pressure for the gene or its expression vector during the extended period of growth. One aspect of the present invention is using an antibiotic that is not inactivated or degraded during growth of the expression host cell as is found with ampicillin. One such preferred embodiment involves the expression of genes encoding resistance to kanamycin as the selective phenotype for maintaining the expression vector which comprises such kanamycin resistance genetic sequences. Expression of large AT-rich clostridial genes in *E. coli* at levels (> 100 mg/liter) provided for by methods of the present invention was hitherto unknown.

Terms as used herein are based upon their art recognized meaning and should be clearly understood by the ordinary skilled artisan.

An immunogenic composition is any composition of material that elicits an immune response in a mammalian host when the immunogenic composition is injected or otherwise introduced. The immune response may be humoral, cellular, or both.

A fusion protein is a recombinant protein encoded by a gene or fragment of a gene, genetically fused to another gene or fragment of a gene.

A booster effect refers to an increased immune response to an immunogenic composition upon subsequent exposure of the mammalian host to the same immunogenic composition. A humoral response results in the production of antibodies by the mammalian host upon exposure to the immunogenic composition.

*r*ARU is a recombinant protein containing the repeating units of *Clostridium difficile* toxin A as defined by Dove *et al.* (Dove et al. Infect. Immun. 58:480-488 (1990)). The nucleotide sequence encoding *r*ARU and the amino acid sequence of *r*ARU are shown in Figs. 2 and 3, respectively. The *r*ARU expressed by pRSETB-ARU-Km^{r} contains the entire repeating units region of toxin A. The invention contemplates the production of this recombinant protein component, or any other protein component containing the entire repeating units of toxin A or any fragment therein, whether expressed alone or as a fusion protein.

Similar methods may be used to isolate, clone and express a recombinant protein component comprising the repeating units of *Clostridium difficile* toxin B (*r*BRU). The nucleotide sequence encoding *r*BRU and the amino acid sequence of *r*BRU are shown in Figs. 6 and 7. respectively. The rBRU expressed by pRSETC-BRU-Km^{r} contains the entire repeating units region of toxin B (see Fig. 8).

The present methods provide for the production of rARU, rBRU or fusion proteins therof which can be used for the preparation of immunogenic compositions comprising rARU or rBRU or both, which are useful as vaccines. Immunogenic compositions may be formulated as vaccines in a pharmaceutically acceptable carrier or diluent (e.g., water, a saline solution (e.g., phosphate-buffered saline), a bicarbonate solution (e.g., 0.24 M NaHCO.sub.3), a suppository, cream, or jelly), which are selected on the basis of the mode and route of administration, and standard pharmaceutical practice, see: U.S. Patent 5,919.463 Thomas, et al., (1999). Suitable pharmaceutical carriers and diluents, as well as pharmaceutical necessities for their use in pharmaceutical formulations, are described in Remington's Pharmaceutical Sciences (Alfonso Gennaro et al., eds.. 17th edn., Mack Publishing Co.. Easton Pa.. 1985), a standard reference text in this field, in the USP/NF, and by Lachman et al. (The Theory & Practice of Industrial Pharmacy, 2nd edn., Lea & Febiger, Philadelphia Pa., 1976). In the case of rectal and vaginal administration, the vaccines are administered using methods and carriers standardly used in administering pharmaceutical materials to these regions. For example, suppositories, creams (e.g., cocoa butter), or jellies, as well as standard vaginal applicators, droppers, syringes, or enemas may be used, as determined to be appropriate by one skilled in the art.

The described vaccine compositions may be administered by any route clinically indicated, such as by application to the surface of mucosal membranes (including: intranasal, oral, ocular, gastrointestinal, rectal, vaginal or genito-urinary). Alternatively, parenteral (e.g., intravenous, subcutaneous, intraperitoneal, or intramuscular) modes of administration may also be used. The amounts of vaccine administered depend on the particular vaccine antigen and any adjuvant employed: the mode and frequency of administration; and the desired effect (e.g., protection and/or treatment), as determined by one skilled in the art. In general, the vaccines will be administered in amounts ranging between 1 µg and 100 mg. Administration is repeated as is determined to be necessary by one skilled in the art. For example, a priming dose may be followed by 3 booster doses at weekly intervals.

Having now generally described the invention, the same will be more readily understood through reference to the following examples which are provided by way of illustration, and are not intended to be limiting of the present invention, unless specified.

### EXAMPLES

### EXAMPLE 1

### Construction of rARU expression vector.

The vector pRSETB-ARU-Km^{r} used for expression and purification was constructed using standard techniques for cloning (Sambrook et al., Molecular Cloning: A Laboratory Manual (1989*)).* The nucleotide sequence of the toxin A gene fragment encoding rARU was derived from the cloned toxin A gene (Dove et al., Infect. Immun. 58:480-488 (1990); Phelps et al.. Infect Immun. 59:150-153 (1991)) and is shown in Fig. 2. The gene fragment encodes a protein 867 amino acids in length (Fig. 3) with a calculated molecular weight of 98 kDa. The gene fragment was subcloned to the expression vector pRSETB. A kanamycin resistance gene was subsequently subcloned to the vector. The resulting vector pRSETB-ARU-Km^{r} expresses *r*ARU. An additional 31 amino acids at the N-terminus of the recombinant protein are contributed by the expression vector pRSETB. The final calculated molecular weight of the recombinant protein is 102 kDa.

### EXAMPLE 2

### Expression and purification of rARU.

*Escherichia coli* T7 expression host strain BL21(DE3) was transformed with pRSETB-ARU-Km^{r} as described (Sambrook et al. Molecular Cloning: A Laboratory Manual (1989)). One liter cultures were inoculated with 10 ml of overnight growth of *Escherichia coli* BL21(DE3) containing pRSETB-ARU-Km^{r} and grown at 37°C in Terrific broth (Sigma, St. Louis, MO) containing 25 µg/ml of kanamycin to an O.D. 600 of 1.8-2.0 and isopropyl B-D-thiogalactopyranoside (IPTG) was added to a final concentration of 40 µM. Cells were harvested after 22 h of induction, suspended in 0.1 liter of standard phosphate buffered saline, pH 7.4. containing 0.2 % casamino acids, and disrupted by sonication. Cellular debris was removed from the lysate by centrifugation. Lysates typically contained a titer (reciprocal of the highest dilution with an *A*₄₅₀ greater than 0.2) of 10⁶ in the TOX-A test EIA (TechLab, Inc., Blacksburg, VA). Lysates were saturated with 40% ammonium sulfate, stirred at 4°C overnight and precipitating proteins were harvested by centrifugation. The ammonium sulfate fraction was suspended in 0.1 liters of 5 mM K₂PO₄. 0.1 M NaCl₂, pH 8.0 and dialyzed extensively against the same buffer at 4°C. Insoluble material was removed by centrifugation. The dialyzed solution was passed through a column containing Sepharose CL-6B chromatography media (50 ml media/100 ml solution). Fractions were collected and monitored for the presence of *r*ARU by EIA using the TOX-A test. Fractions containing EIA activity were analyzed by SDS-PAGE for the presence *o*f rARU at a molecular weight of approximately 102 kDa. Fractions containing a single band of *r*ARU were pooled. To further ensure purity the pooled solution was again passed over a Sepharose CL-6B column (25 ml media/100 ml protein solution). The solution containing purified *r*ARU was filtered sterilized by passage through a 22 µ filter and stored at 4°C. Purified *r*ARU along with samples from the steps of purification (lysate and dialyzed ammonium sulfate fraction) are shown in Fig. 5. The procedure typically yields approximately 100 mg *r*ARU per liter of *E. coli*/pRSETB-ARU-Km^{r} culture. A combined 6-liter batch yielded 0.850 liters of *r*ARU at 0.88 mg/ml for a total of 748 mg of *r*ARU or 125 mg/liter of culture. The amount of *r*ARU recovered represented 23% of the total soluble protein.

### EXAMPLE 3

### Construction of rBRU expression vector.

The vector pRSETC-BRU-Km^{r} used for expression and purification was constructed using standard techniques for cloning (Sambrook et al.. Molecular Cloning: A Laboratory Manual (1989*)).* The nucleotide sequence of the toxin B gene fragment encoding *r*BRU was derived from the cloned toxin B gene (Barroso et al., Nucleic Acids Res 18:4004 (1990)) and is shown in Fig. 6. The gene fragment encodes a protein 622 amino acids in length with a molecular weight of approximately 70 kDa. The gene fragment was subcloned to the expression vector pRSETC. A kanamycin resistance gene was subsequently subcloned to the vector. The resulting vector pRSETC-BRU-Km' expresses *r*BRU.

### EXAMPLE 4

### High-level expression and partial purification of rBRU.

One liter of *Escherichia coli* pRSETC-BRU-Km^{r} was grown for 25 h at 37°C in a shaking incubator. Cells were harvested by centrifugation and resuspended in 0.1 liter phosphate buffered saline with 0.2% casamino acids. Supernatant of the culture at harvest had a pH of 6.2. Cells were disrupted by sonication and cellular debri was removed by centrifugation. The 10X lysate is shown in Fig. 9. lane 3.

### EXAMPLE 5.

### Immune response to the rARU component of the conjugates.

Antibodies to *C*. *difficile* toxin A (CDTA). Antibodies to native toxin A were measured by ELISA. with toxin A isolated from C. *difficile* as the coating antigen, and by in-vitro neutralization of cytotoxicity (Lyerly et al. Infect. Immun. 35:1147-1150 (1982)). Human intestinal epithelial HT-29 cells (ATCC HTB 38) were maintained in 96 well plates with McCoy's 5A medium supplemented with 10% fetal calf serum in a 5% CO₂ atmosphere. HT-29 cells were chosen because of their high sensitivity to CDTA probably because of the high density of the carbohydrate receptor on their surface. Serial 2-fold dilutions of sera were incubated with 0.4 µg/ml of CDTA for 30 min at room temperature. CDTA-serum mixtures were added to the wells at a final concentration of 20 ng of toxin A per well (about 200 times the minimal cytotoxic dose for HT-29 cells) in a final volume of 0.2 ml. The neutralization titer is expressed as the reciprocal of the highest dilution that

**TABLE 1. Serum antibodies (µg/ml) to Clostridium difficile toxin A (CDTA) elicited in mice by recombinant enterotoxin A (rARU) or polysaccharides bound to rARU alone or succinylated (rARUsucc)**

| | | ELISA (Geometric mean and 25-75 centiles) | | |
|---|---|---|---|---|
| Conjugate | µg *r*ARU Injected | First injection | Second injection | Third injection |
| | | | | |
| *r*ARU* | 6.94 | ND | ND | 717 (621-863) |
| Pn14-*r*ARU | 1.29 | 3.70 (2.55-5.08) | 80.1 (69.8-131) | 194 (113-236) |
| Pn14*r*ARU *succ* | 7.30 | 7.94 (5.21-11.3) | 183 (146-175) | 371 (274-463) |
| SF-*r*ARU | 3.90 | ND | 433 (258-609) | 613 (485-778) |
| SF-*r*ARUsucc | 6.94 | ND | 191 (118-291) | 518 (366-615) |
| SF-*r*ARU* | 3.90 | ND | ND | 437 (372-547) |
| SF-*r*ARUsucc* | 6.94 | ND | ND | 242 (172-443) |
| K1 | 8.08 | 10.7 (6.75-17.2) | 84.9 (72.5-131) | 390 (279-470) |

| | | | | |
|---|---|---|---|---|
| 183 vs 7.94 p=0.0001, 371 vs 183 p=0.0005. 80.1 vs 3.70 p=0.0001, 194 vs 80.1 p=0.007, 7.94 vs 3.70 p=0.01, 183 vs 80.1 p=0.004, 371 vs 194 p=0.01 *hsd/ICR mice. Remainder were NIH SA mice. ND (not done). 6 wks-old mice were injected SC with 2.5 µg of polysaccharide as a conjugate at 2 wk intervals. Groups of mice (n=10) were exsanguinated 7 days after each injection and their sera assayed for anti-CDTA by ELISA. completely neutralized cytotoxicity. | | | | |

All 5 conjugates elicited high levels of anti-CDTA (194-613 µg/ml) (Table 1). Since the 2.5 µg immunizing dose of the conjugates was based on its polysaccharide content, the amount of *r*ARU injected was different for each conjugate. For example, on a protein weight basis. Pn14-rARU. with 1.29 µg of *r*ARU. elicited 194 µg CDTA antibody/ml (130.3 µg Ab/µg rARU injected). In contrast. Pn14-*r*ARU*succ.* that contained 7.3 µg of *r*ARU per dose, elicited 371 µg CDTA antibody/ml (50.8 µg Ab/µg *r*ARU*succ* injected). Pn14-*r*ARU induced more anti-CDTA per µg *r*ARU than Pn14-*r*ARU*succ*. however, the total amount of anti-CDTA elicited by Pn14-*r*ARU*succ* was greater due to its higher content of *r*ARU. The difference between the levels of anti-CDTA elicited by Pn14-*r*ARU (194 µg CDTA antibody/ml) compared with Pn14-*r*ARU*succ* (371 µg CDTA antibody/ml) was significant.

SF-*r*ARU, containing 3.9 µg of *r*ARU. elicited 437 µg CDTA antibody/ml (112.0 µg Ab/µg *r*ARU injected) compared to 518 µg CDTA antibody/ml for SF-*r*ARU*succ* (34.9 µg Ab/µg *r*ARU*succ* injected). Although the specific immunogenic activity for the *r*ARU*succ* was lower than that of the *r*ARU in the SF conjugates, there was no statistical difference between the levels of CDTA antibody elicited by the two conjugates (437 µg Ab/ml for SF-*r*ARU*succ* vs 242 µg Ab/ml for SF-*r*ARU).

K1-*r*ARU*succ*, that elicited 390 µg CDTA antibody/ml, had comparable specific immunogenic activity of its *r*ARU component (48 µg Ab/ml per µg *r*ARU*succ*).

### EXAMPLE 6

### CDTA neutralizing antibodies.

Individual sera obtained 7 days after the third injection of the conjugates were assayed individually for their neutralization of approximately 200 times the cytotoxic dose of CDTA on human intestinal epithelial HT-29 cells. All sera from the mice immunized with the conjugates had a neutralizing titer greater than or equal to 64. The geometric mean and range of neutralizing titers for each conjugate is shown in Table 2.

**TABLE 2. Serum neutralizing activity against the in vitro cytotocicity for HT-29 cells of Clostridium difficile toxin A (CDTA)**

| Immunogen | µg Ab/ml (ELISA) | Reciprocol neutra;ization titer (GM and range) | |
|---|---|---|---|
| Pn14-*r*ARU | 194 | 104 | 64-256 |
| Pn14-*r*ARU*succ* | 371 | 111 | 64-128 |
| SF-*r*ARU | 613 | 194 | 64-256 |

| | | | |
|---|---|---|---|
| Neutralizing titers were the highest serum dilution that completely inhibited the cytotoxicity of CDTA (20 ng/well) on HT-29 cells. The titers represent the geometric mean of sera from general purpose Swiss Albino mice (n=10) obtained 7 days after the 3rd injection. Anti-CDTA was measued by ELISA and the mean value expressed as µg Ab/ml serum. * Affinity purified goat antibody | | | |

### EXAMPLE 7

### Protection against lethal challenge with CDTA (Table 3).

Hsd/ICR mice were injected with SF-*r*ARU, SF-*r*ARU*succ* or *r*ARU as described in EXAMPLE 4 above. One week after the third injection, the mice were challenged intraperitoneally with a lethal dose (150 ng) of CDTA. Almost all mice vaccinated with either conjugate or *r*ARU were protected. Based upon the amount of *r*ARU injected. *r*ARU and SF-*r*ARU elicited similar levels of anti-CDTA. As expected. SF-*r*ARU*succ* elicited lower levels of anti-CDTA than the other two immunogens but the recipients were comparably protected.

Conjugate-induced antibody levels approached or surpassed the neutralizing activity of an affinity-purified goat antibody, containing 0.5 mg/ml. that was raised against formalin inactivated CDTA.

**TABLE 3. Protection of mice against lethal challenge with 150 ng of Clostridium difficile toxin A (CDTA) a induced by vaccination with polysaccharide-rARU conjugates**

| Immunogen | µg *r*ARU injected | Survivals /total | CDTA antibodies (ELISA)*^{b}* | Reciprocal neutralization titer*^{c}* |
|---|---|---|---|---|
| *r*ARU | 6.94 | 19/20 | 717 (621-863) | 128-256 |
| SF-*r*ARU | 3.90 | 17/20 | 437 (372-547) | 1 28-256 |
| SF-*r*ARU*succ* | 6.94 | 19/20 | 242 (172-443) | 64-256 |
| PBS | 0 | 2/15 | Not determined | <2 |

| | | | | |
|---|---|---|---|---|
| *a* Mice (hsd/ICR) injected I.P. with 150 ng of CDTA 7 days after the 3rd injection of *r*ARU or conjugate. *b* Mean antibody level (25-75 centiles) of sera used for pool (n=10 from each group bled 4 h before challenge with CDTA. c Highest dilutions of sera (range) that completely neutralized the cytotoxicity of CDTA (20 ng/well) on HT-29 cells. | | | | |

This invention has been described by a direct description and by examples. As noted above, the examples are meant to be only examples and not to limit the invention in any meaningful way.

### LITERATURE CITED

U.S. Pat. No. 5,098,826 (Wilkins et al.) (1992).
U.S. Pat. No. 5,736,139 (Kink et al.) (1998)
U.S. Pat. No. 5.919.463 (Thomas et al.) (1999)
Lyerly, D.M. and T.D. Wilkins, in Infections of the Gastrointestinal Tract, Chapter 58, pages 867-891. Raven Press, Ltd. New York 1995
Moncrief et al.. Infect. Immun. 65:1105-1108 (1997);
Barroso et al., Nucl. Acids Res. 18:4004 (1990);
Dove et al. Infect. Immun. 58:480-488 (1990)). (
Krivan et al.. Infect. Immun. 53:573-581 (1986);
Tucker. K. and T.D. Wilkins, Infect. Immun. 59:73-78 (1991)).
Just et al. Nature 375:500-503 (1995).
Just et al. J. Biol. Chem 270:13932-13939 (1995)).
Hofmann et al. J. Biol. Chem. 272:11074-11078 (1997),
Faust and Song, Biochem. Biophys. Res. Commun. 251:100-105 (1998))
Lyerly et al. Current Microbiology 21:29-32 (1990)

## Claims

1. A method to produce the repeating unit portion of *Clostridium difficile* toxin A (*r*ARU) or toxin B (*r*BRU) or a fusion protein thereof in high yield in *E*. *coli* bacteria which method comprises
(i) culturing bacteria which have been modified with a nucleic acid comprising
(a) an expression system wherein a nucleotide sequence encoding said *r*ARU or *r*BRU or a fusion protein thereof is operably linked to an inducible promoter; and
(b) a nucleotide sequence encoding resistance to kanamycin,
until completion of the exponential growth phase; and
(ii) inducing said promoter to moderate levels over a period of 20-24 hours of post-exponential growth;
wherein constant selective pressure for said nucleotide sequence encoding resistance to kanamycin is maintained during the extended period of growth, and whereby said *r*ARU or *r*BRU or a fusion protein thereof is produced at levels greater than 50 milligrams per liter of culture.

2. The method of claim 1, wherein said rARU or *r*BRU or a fusion protein thereof is produced at levels greater than 100 mg/l.

3. The method of claim 1 or 2, wherein the inducible promoter is the T7 promoter.

4. The method of any one of claims 1-3, which further comprises recovering said *r*ARU or *r*BRU or fusion protein from the culture.

## Patentansprüche

1. Verfahren zur Herstellung des Wiederholeinheit-Teils des Toxins A (*r*ARU) oder des Toxins B (*r*BRU) von *Clostridium difficile* oder eines Fusionsproteins davon in hohen Ausbeuten in *E. col*i-Bakterien, welches die folgenden Schritte umfasst:
(i) das Kultivieren von Bakterien, welche mit einer Nucleinsäure modifiziert wurden, welche Folgendes umfasst
(a) ein Expressionssystem, in dem eine Nucleotidsequenz, welche *r*ARU oder *r*BRU oder ein Fusionsprotein davon codiert, funktionell verknüpft ist mit einem induzierbaren Promotor; und
(b) eine Nucleotidsequenz, welche eine Kanamycinresistenz codiert, bis zur Vervollständigung der exponentiellen Wachstumsphase; und
(ii) Induktion des Promotors auf moderatem Niveau über einen Zeitraum von 20-24 Stunden des post-exponentiellen Wachstums;
wobei während der verlängerten Wachstumsphase ein konstanter Selektionsdruck für die Nucleotidsequenz aufrecht erhalten wird, welche die Kanamycinresistenz codiert, und wobei *r*ARU oder *r*BRU oder ein Fusionsprotein davon in Mengen von mehr als 50 Milligramm pro Liter Kultur produziert wird.

2. Verfahren nach Anspruch 1, wobei *r*ARU oder *r*BRU oder ein Fusionsprotein davon in Mengen von mehr als 100 mg/l produziert wird.

3. Verfahren nach Anspruch 1 oder 2, wobei der induzierbare Promotor der T7-Promotor ist.

4. Verfahren nach einem der Ansprüche 1 bis 3, welches weiterhin die Gewinnung von *r*ARU oder *r*BRU oder des Fusionsproteins davon aus der Kultur umfasst.

## Revendications

1. Méthode pour produire la portion d'unités répétitives de la toxine A (*r*ARU) ou de la toxine B (*r*BRU) de *Clostridium difficile* ou d'une protéine de fusion de celle-ci avec un rendement élevé dans des bactéries E. coli, ladite méthode comprenant :
(i) la culture de bactéries qui ont été modifiées avec un acide nucléique comprenant
(a) un système d'expression dans lequel une séquence nucléotidique codant pour ladite portion *r*ARU ou *r*BRU ou pour une protéine de fusion de celle-ci est liée de manière opérationnelle à un promoteur inductible ; et
(b) une séquence nucléotidique codant pour la résistance à la kanamycine,
jusqu'à l'achèvement de la phase de croissance exponentielle ; et
(ii) l'induction dudit promoteur à des niveaux modérés pendant 20-24 heures de croissance post-exponentielle ;
dans laquelle une pression sélective constante pour ladite séquence nucléotidique codant pour la résistance à la kanamycine est maintenue pendant la période prolongée de croissance, et par laquelle ladite *r*ARU ou *r*BRU ou une protéine de fusion de celle-ci est produite à un niveau supérieur à 50 milligrammes par litre de culture.

2. Méthode de la revendication 1, dans laquelle ladite *r*ARU ou *r*BRU ou une protéine de fusion de celle-ci est produite à des niveaux supérieurs à 100 mg/l.

3. Méthode de la revendication 1 ou 2, dans laquelle le promoteur inductible est le promoteur T7.

4. Méthode de l'une quelconque des revendications 1 à 3, comprenant en outre la récupération de ladite *r*ARU ou *r*BRU ou d'une protéine de fusion de celle-ci, de la culture.
